(19) Europäisches Patentamt
European Patent Office
Office européen des brevets

(11) Veröffentlichungsnummer: **0 148 440**
**B1**

(12) # EUROPÄISCHE PATENTSCHRIFT

(45) Veröffentlichungstag der Patentschrift: **05.10.88**

(51) Int. Cl.[4]: **C 07 D 209/90,** A 61 K 31/40

(21) Anmeldenummer: **84115218.4**

(22) Anmeldetag: **12.12.84**

(54) **1,3,4,5-Tetrahydrobenz[c,d]indole, ein Verfahren zu ihrer Herstellung und ihre Verwendung.**

(30) Priorität: **23.12.83 DE 3346573**

(43) Veröffentlichungstag der Anmeldung:
**17.07.85 Patentblatt 85/29**

(45) Bekanntmachung des Hinweises auf die Patenterteilung:
**05.10.88 Patentblatt 88/40**

(84) Benannte Vertragsstaaten:
**AT BE CH DE FR GB IT LI NL SE**

(56) Entgegenhaltungen:
**EP - A - 0 002 570**
**EP - A - 0 029 581**
**EP - A - 0 091 328**
**EP - A - 0 153 083**
**EP - A - 0 162 695**
**CH - A - 517 732**
**US - A - 3 336 307**

**E. SCHRÖDER, C. RUFER, R. SCHMIECHEN, Arzneimittelchemie I, Verlag Thieme, Stuttgart 1976, S. 26**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG, Berliner Strasse 156, D-5000 Köln 80 (DE)**

(72) Erfinder: **Glaser, Thomas, Dr., Rybnikerstrasse 6, D-5000 Köln 80 (DE)**
Erfinder: **Junge, Bodo, Dr., Spiekern 23, D-5600 Wuppertal 23 (DE)**
Erfinder: **Traber, Jörg, Dr., Löwenburgstrasse 12, D-5204 Lohmar 31 (DE)**
Erfinder: **Allen, George, Prof., Vanderbilt University, Nashville/Tennessee 37232 (US)**

(74) Vertreter: **Adrian, Albert, Dr. et al, c/o BAYER AG Konzernverwaltung RP Patentabteilung, D-5090 Leverkusen 1, Bayerwerk (DE)**

**EP 0 148 440 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 1,3,4,5-Tetrahydrobenz[c,d]indole und deren Salze mit physiologisch unbedenklichen Säuren, ein Verfahren zu ihrer Herstellung sowie ihre Verwendung bei der Bekämpfung von Krankheiten. Insbesondere betrifft die Erfindung die Verwendung der 1,3,4,5-Tetrahydrobenz[c,d]indole zur Behandlung von kardiovaskulären Erkrankungen und Erkrankungen des zentralen Nervensystems durch eine Wirkung auf das serotoninerge System.

Die Erfindung betrifft Tetrahydrobenzindole der allgemeinen Formel I

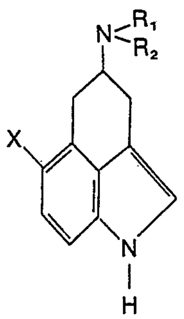

I

in welcher

$R_1$ und $R_2$ Wasserstoff oder $C_1$–$C_6$-Alkyl bedeuten oder zusammen mit dem Stickstoffatom einen gebebenenfalls durch 1 oder 2 $C_1$–$C_6$-Alkylgruppen substituierten heterocyclischen 5- oder 6-Ring bilden,

X für $OR_3$ oder $SR_3$ steht, wobei

$R_3$ Wasserstoff oder $C_1$–$C_4$-Alkyl bedeutet,

sowie deren Salze.

Die vorliegende Erfindung betrifft sowohl Wirkstoffe der Formel I in racemischer Form als auch in Form der einzelnen Enantiomeren.

Die erfindungsgemässen Tetrahydrobenzindole können hergestellt werden, indem man Verbindungen der allgemeinen Formel II

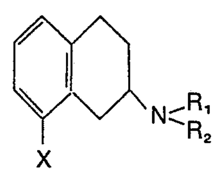

(II)

in der

$R_1$, $R_2$ und X die obengenannte Bedeutung haben, in para-Stellung zum Substituenten X nitriert, die entstandene Nitroverbindung mit Oxalsäureestern der Formel $R_4$–O–CO–CO–$OR_4$, wobei

$R_3$ $CH_3$ oder $C_2H_5$ bedeutet,

zu Verbindungen der allgemeinen Formel III

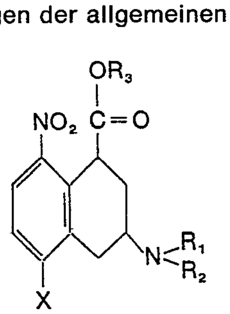

(III)

in der

$R_1$, $R_2$, $R_3$ und X die obengenannte Bedeutung haben, acyliert,

die Estergruppe verseift, die Nitrogruppe reduziert und anschliessend in einem oder mehreren Schritten cyclisiert und decarboxyliert.

Weiterhin kann man die erfindungsgemässen Tetrahydrobenzindole herstellen, indem man Verbindungen der allgemeinen Formel II

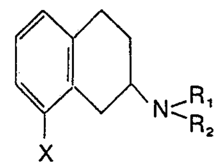

(II),

in der

$R_1$, $R_2$ und X die obengenannte Bedeutung haben, in para-Stellung zum Substituenten X nitriert, zu den entsprechenden Aminen reduziert, dann in die Isonitrile der Formel

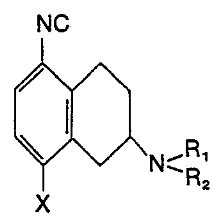

(IV),

in der

$R_1$, $R_2$ und X die obengenannte Bedeutung haben, überführt und diese in Gegenwart einer starken Base cyclisiert.

Die Herstellung der Isonitrile kann beispielsweise in einem Zweiphasensystem mit $CHCl_3$/NaOH erfolgen.

Die Cyclisierung mit starken Basen kann beispielsweise mit Lithiumdiisopropylamid erfolgen.

Die erfindungsgemässen Wirkstoffe der Formel I unterscheiden sich von bekannten Wirkstoffen ähnlicher Struktur (z.B. US PS 4 110 339) eindeutig durch den Substituenten X in 6-Stellung des tricyclischen Ringsystems. Während die bekannten Verbindungen, in denen X Wasserstoff bedeutet, eine starke Wirkung auf das dopaminerge System aufweisen, tritt bei den erfindungsgemässen Verbindungen der Formel I, in denen X die oben gegebene Bedeutung hat, die Wirkung auf das dopaminerge System gegenüber einer starken Wirkung auf das serotoninerge System in den Hintergrund. Es konnte gezeigt werden, dass die erfindungsgemässen Verbindungen eine hohe Affinität zu Serotoninrezeptoren des Gehirns aufweisen, insbesondere zu denen des 5-$HT_1$-Typs. Die erfindungsgemässen Verbindungen sind somit selektive 5-$HT_1$-Rezeptor-Liganden.

Die erfindungsgemässen Wirkstoffe haben im Tierversuch starke Wirkung auf das zentrale Nervensystem. Insbesondere zeigen sie Wirkung in Versuchsanordnungen, in denen anxiolytische und antidepressive Wirkungen gefunden werden. Darüber hinaus führen sie zu einer starken Stimulierung des Sexualverhaltens bei Ratten.

Aufgrund dieser Eigenschaften können die erfindungsgemässen Verbindungen in Arzneimitteln

verwendet werden. Sie eignen sich zur Bekämpfung von Erkrankungen des zentralen Nervensystems, insbesondere zur Behandlung von Angst und Spannungszuständen, Schlafstörungen und Depressionen, vorzugsweise zur Behandlung von Problemen der sexuellen Potenz.

Bevorzugt sind Verbindungen der Formel I, in denen

$R_1$ und $R_2$ für einen geradkettigen oder verzweigten $C_1$–$C_4$-Alkylrest stehen oder $R_1$ und $R_2$ zusammen mit dem Stickstoffatom einen Pyrrolidin- Piperidin-, 2,5-Dimethylpyrrolidin- oder 2,6-Dimethylpiperidinrest bilden und

X für $OCH_3$ oder $SCH_3$ steht,
sowie deren Salze.

Besonders bevorzugt sind Verbindungen der Formel I, in denen

$R_1$ und $R_2$ einen geradkettigen oder verzweigten $C_1$–$C_4$-Alkylrest und

X $OCH_3$ bedeutet,
sowie deren Salze.

Die Herstellung der erfindungsgemässen Tetrahydrobenzindole sei beispielsweise durch das folgende Formelschema erläutert:

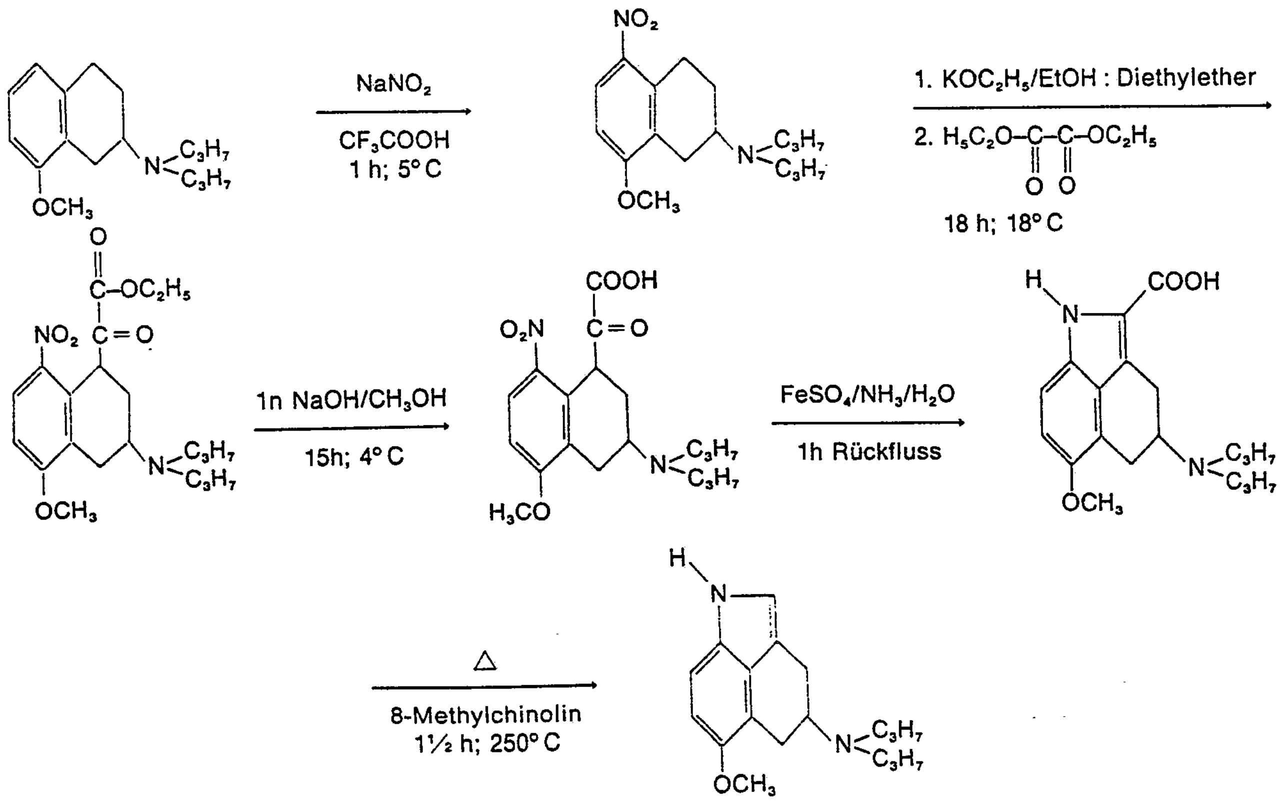


Die Nitrierung der 2-Aminotetraline II zu den Nitroverbindungen III erfolgt in Mineralsäure oder organischen Carbonsäuren mit konz. oder rauchender Salpetersäure oder $NaNO_3$ als Nitrierungsreagenz. Bevorzugt werden Schwefelsäure, Salpetersäure oder Trifluoressigsäure als Lösungsmittel verwendet. Die Reaktion wird bei Temperaturen zwischen $-60\,°C$ und $+40\,°C$ durchgeführt. Der bevorzugte Temperaturbereich liegt zwischen $-20\,°C$ und Raumtemperatur.

Die Acylierung der Nitroverbindungen zu den Verbindungen III mit Oxalsäuredimethylester oder -diethylester wird in Gegenwart einer starken Base in einem inerten protischen oder aprotischen Lösungsmittel durchgeführt. Als Basen zur Deprotonierung der aktivierten Methylengruppe kommen metallorganische Basen, wie Lithiumdiisopropylamid, n-Butyllithium, NaH oder Alkalialkoholate, wie Na- oder K-Methylat oder -ethylat, in Betracht.

Als Lösungsmittel werden aprotische Lösungsmittel, insbesondere Ether, wie Diethylether, Tetrahydrofuran, Dioxan, 1,2-Dimethoxyethan, Diglyme oder aprotische Lösungsmittel, wie Dimethylformamid oder Dimethylsulfoxid oder protische Lösungsmittel, insbesonder Alkohole, wie Methanol oder Ethanol, oder Gemische dieser Lösungsmittel verwendet.

Die Reaktion wird bei Temperaturen zwischen $-80\,°C$ und Raumtemperatur durchgeführt. Eine bevorzugte Ausführungsform der Acylierungsreaktion ist die Verwendung von Oxalsäurediethylester als Acylierungsreagenz, Kaliumethanolat als Base, einem Gemisch von Diethylether/Ethanol als Lösungsmittel und die Durchführung der Reaktion bei Raumtemperatur.

Die Verseifung der Esterfunktion in den Verbindungen der Formel III wird bevorzugt in einem wässrig alkalischen Milieu durchgeführt. Als Basen werden vorzugsweise Alkali- oder Erdalkalihydroxide oder -carbonate verwendet. Die Reaktion wird in Wasser oder bevorzugt in einem Lösungsmittelgemisch aus Wasser und einem mit Wasser mischbaren Lösungsmittel, wie MeOH oder EtOH, durchgeführt. Die Reaktionstemperatur liegt zwischen 0 °C und Raumtemperatur.

Die Reduktion oder Nitrogruppe in den Verbindungen der Formel III und der Ringschluss zu den Verbindungen der Formel V

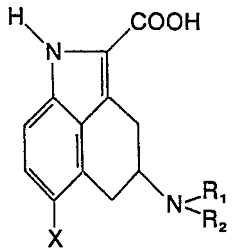

(V),

in der

$R^1$, $R^2$ und X die obengenannte Bedeutung haben,

erfolgt unter Bedingungen, die für die Reduktion einer aromatischen Nitrogruppe zu einer $NH_2$-Gruppe gebräuchlich sind. Als Reduktionsmittel lassen sich beispielsweise verwenden: mit Edelmetallkatalysatoren, wie Pt oder Pd aktivierter Wasserstoff, Eisen-II-Salze in alkalischem, z.B. ammoniakalischem Medium; Zink in Eisessig oder Natriumdithionit. Als Lösungsmittel wird vorzugsweise Wasser oder ein Alkohol/Wassergemisch oder Eisessig verwendet. Die Reaktionstemperaturen liegen zwischen Raumtemperatur und der Siedetemperatur des Lösungsmittels. Bevorzugt wird die Reduktion mit Eisen-II-sulfat in einer wässrig ammoniakalischen Lösung bei Rückflusstemperatur durchgeführt.

Die Decarboxylierung der Verbindungen V zu den erfindungsgemässen Verbindungen der Formel I wird in einem inerten Lösungsmittel bei Temperaturen zwischen 150 und 300 °C, gegebenenfalls in Gegenwart eines Katalysators wie Cu-Pulver durchgeführt. Bevorzugt finden basische organische Lösungsmittel, wie Chinolin, Chinaldin oder 8-Methylchinolin, Verwendung.

Die Ausgangsstoffe der Formel II sind literaturbekannt oder können in Analogie zu den bekannten Verbindungen hergestellt werden (J. Chem. Soc. 1965, 2636).

Die Verbindungen der Formel III, IV und V sind neu. Ihre Herstellung und ihre Verwendung zur Herstellung der erfindungsgemässen Verbindungen der Formel I sind ein wesentlicher Bestandteil dieser Erfindung.

Zur vorliegenden Erfindung gehören pharmazeutische Zubereitungen, die neben nicht-toxischen, inerten, pharmazeutisch geeigneten Trägerstoffe eine oder mehrere der erfindungsgemässen Verbindungen oder deren Salze enthalten oder die aus einer oder mehrerer der erfindungsgemässen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1, 2, 3 und 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel eine Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzukker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin, und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit, und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opalisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakofett, und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können ausser Verbindungen der Formel (I) und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere oral und intravenös appliziert werden.

Im allgemeinen hat es sich als vorteilhaft erwiesen, den oder die Wirkstoffe bei parenteraler (i.v. oder i.m.) Applikation in Mengen von etwa 0,005 bis etwa 5, vorzugsweise von 0,01 bis 1 mg/kg Körpergewicht je 24 Stunden und bei oraler Applikation in Mengen von etwa 0,01 bis etwa 10, vorzugsweise 0,05 bis 5 mg/kg Körpergewicht je 24 Stunden, gegebenenfalls in Form mehrerer Einzelgaben und Erzielung der gewünschten Ergebnisse zu verabreichen. Eine Einzelgabe enthält den oder die Wirkstoffe, vorzugsweise in Mengen von etwa 0,005 bis etwa 10, insbesondere 0,01 bis 1 mg/kg Körpergewicht.

Es kann jedoch erforderlich sein, von den genannten Dosierungen abzuweichen, und zwar in Abhängigkeit von der Art und dem Körpergewicht des zu behandelnden Objekts, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben aufgeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Zur vorliegenden Erfindung gehören auch solche Arzneimittel, die neben Verbindungen der Formel (I) noch weitere Wirkstoffe enthalten. Vorzugsweise seien genannt:

β-Rezeptorenblocker, Parasympathikolytika, Anxiolytika, Neuroleptika, Hypnotika und Tranquilizer.

Beispiel 1
6-Methoxy-4-dipropylamino-1,3,4,5-tetrahydrobenz[c,d]indol

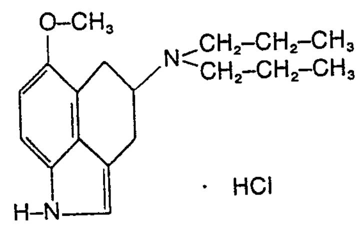

Eine Lösung von 0,35 g (0,00106 Mol)
6-Methoxy-4-dipropylamino-1,3,4,5-tetrahydrobenz[c,d]indol-2-carbonsäure
in 10 ml 8-Methylchinolin wurde mit 0,2 g Cu-Pulver versetzt und anschliessend unter Stickstoffspülung im Custilator 1½ h auf 250 °C erhitzt. Die dunkelrotgefärbte Reaktionslösung wurde auf eine mit Kieselgel (Kieselgel 60 der Fa. E. Merck, Darmstadt) gefüllte Säule gegeben. Zur Abtrennung von 8-Methylchinolin wurde mit $CH_2Cl_2$ eluiert und anschliessend mit $CH_2Cl_2$ : MeOH / 20 : 4 das Reaktionsprodukt. Die Rohsubstanz wurde erneut an Kieselgel (Kieselgel 60 der Fa. E. Merck, Darmstadt) mit Essigester/MeOH/20 : 2 chromatografiert. Das Reaktionsprodukt wurde in Ether gelöst und durch Zugabe von etherischer Salzsäure ins Hydrochlorid überführt. Das Hydrochlorid wurde im Exsikkator über NaOH getrocknet.

Ausbeute: 0,226 g. Fp. 134°–36 °C.

Herstellung der Ausgangsverbindungen:
6-Methyl-4-dipropylamino-1,3,4,5-tetrahydrobenz[c,d]indol-2-carbonsäure

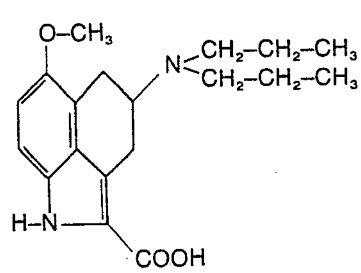

Eine Lösung von 2 g (0,005285 Mol) 4-(5-Nitro-8-methoxy-2-dipropylamino-1,2,3,4-tetrahydronaphthalin)-2-oxo-essigsäure in 7,5 ml 25%igem wässrigem Ammoniak wurde unter Rühren mit einer warmen Lösung von 9,69 g (0,03485 Mol) $FeSO_4 \cdot 7H_2O$ in 10,6 ml $H_2O$ versetzt und anschliessend 1 h unter Rückfluss erhitzt.

Nach Abkühlung auf Raumtemperatur wurde der unlösliche schwarze Rückstand abfiltriert und mit einem Gemisch aus MeOH : $CH_2Cl_2$/50 : 50 gewaschen. Nach Entfernung des Lösungsmittels wurde der Rückstand an Kieselgel (Kieselgel 60 der Fa. E. Merck, Darmstadt) mit $CH_2Cl_2$ : Me OH/ 20 : 6 chromatografiert. Das so gewonnene Produkt wurde durch Verreiben mit $CH_2Cl_2$ kristallisiert und im Exsikkator über $P_2O_5$ getrocknet.

Ausbeute: 0,35 g.

4-(Nitro-8-methoxy-2-dipropylamino-1,2,3,4-tetrahydronaphthalin)-2-oxoessigsäure

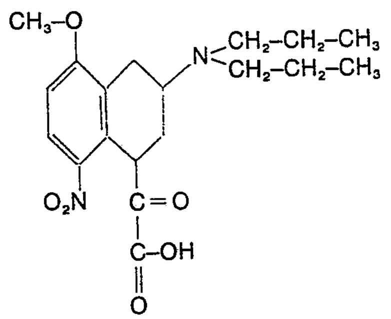

11,5 g (0,0283 Mol) Ethyl-2-(5-nitro-8-methoxy-2-dipropylamino-1,2,3,4-tetrahydronaphthalin)-2-oxoacetat wurden in 300 ml MeOH gelöst und unter Stickstoffspülung bei 18 °C mit 100 ml (0,1 Mol) 1 n NaOH versetzt. Die rotgefärbte Reaktionslösung liess man anschliessend 14 Stunden lang bei +4 °C stehen. Die Reaktionslösung wurde mit 100 ml (0,1 Mol) 1 n HCl neutralisiert und am Rotationsverdampfer von MeOH befreit. Die verbleibende wässrige Phase extrahierte man mit $CH_2Cl_2$. Nach Entfernen des Lösungsmittels trocknete man den Rückstand im Exsikkator über $P_2O_5$.

Ausbeute: 10,2 g gelbgefärbtes kristallines Produkt. Fp (Mettler FP 61) = 200,9 °C.

Ethyl-2-(5-nitro-8-methoxy-2-dipropylamino-1,2,3,4-tetrahydronaphthalin)-2-oxoacetat

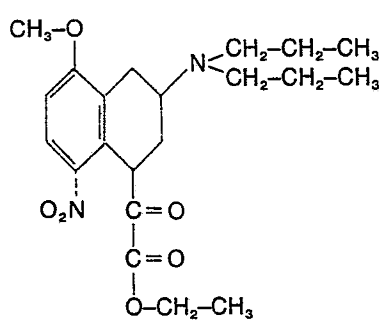

0,94 g (0,024 Mol) Kalium wurde unter Stickstoff und Feuchtigkeitsausschluss mit 22 ml absolutem Diethylether überschichtet und anschliessend unter Rühren bei Raumtemperatur in 5 Portionen mit je 1,11 ml (0,0191 Mol) abs. Ethanol versetzt. Nachdem sich das Kalium gelöst hatte, gab man zur Reaktionslösung 3,26 ml (0,024 Mol) Oxalsäurediethylester und eine Lösung von 7,4 g (0,02415 Mol) 5-Nitro-8-methoxy-2-dipropylamino-1,2,3,4-tetrahydro-naphthalin in 7,5 ml abs. Diethylether zu, liess 1½ Stunden rühren, gab dann den zweiten Teil von 3,26 ml (0,024 Mol) Oxalsäurediethylester zu und vervollständigte die Reaktion durch 18 stündiges Rühren bei 18 °C. Die Reaktionslösung wurde mit 150 ml $CH_2Cl_2$ versetzt und über Kieselgur filtriert. Das Filtrat befreite man vom Lösungsmittel und chromatografierte den öligen Rückstand an Kieselgel (Kieselgel 60 der Fa. E. Merck, Darmstadt; 0,04–0,063 mm Korngrösse) mit dem Laufmittelgemisch $CH_2$-$Cl_2$ : MeOH/ 20 : 0,5.

Ausbeute: 4,3 g; 1,4 g an Ausgangsprodukt wurden zurückgewonnen.

5-Nitro-8-methoxy-2-dipropylamino-1,2,3,4-tetrahydronaphthalin

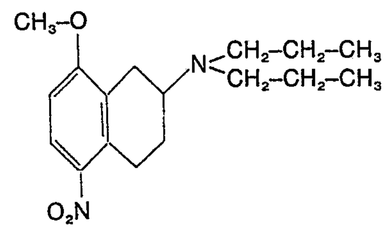

Eine Lösung von 5,5 g (0,02106 Mol) 8-Methoxy-2-dipropylamino-1,2,3,4-tetrahydronaphthalin in 152 ml (1,976 Mol) Trifluoressigsäure wurde unter Rühren, Feuchtigkeitsausschluss und Stickstoffspülung bei einer Temperatur von 5 °C mit 2,9 g (0,0341 Mol) Natriumnitrat versetzt und anschliessend 1 Stunde bei 5 °C gerührt.

Die Reaktionslösung wurde auf Eis gegeben und mit einer 30%igen wässrigen NaOH-Lösung auf pH 10 eingestellt. Anschliessend extrahierte man mit Methylenchlorid. Den Rückstand der $CH_2Cl_2$-Phase chromatografierte man an $Al_2O_3$ ($Al_2O_3$ 90 der Firma Merck, Darmstadt; Korngrösse 0,063–0,200 mm) mit Toluol als Laufmittel.

Ausbeute: 3,3 g gelbgefärbtes Öl (50,8% der Theorie).

Beispiel 2 (Anwendung)

Die Affinität von 6-Methoxy-4-dipropylamino-1,3,4,5-tetrahydrobenz(c,d)indol nach Beispiel 1 zu Serotoninrezeptoren des Gehirns wurde in dem folgenden Affinitätstest (Tab. 1) überprüft. Es zeigt sich eine hohe Bindungsstärke.

Tabelle 1
Affinität zu Serotonin-Rezeptoren

| Rezeptor-Typ | Membran-Material | $^3$H-Ligand | Prüfsubstanz | Inhibitionskonstante $K_i$ (nMol. $1^{-1}$) |
|---|---|---|---|---|
| 5-$HT_1$ | Hippocampus-Kalb | $^3$H-Serotonin | Serotonin | 1 |
| | | | Ketanserin | 2000 |
| | | | Beispiel 1 | 0,8 |
| 5-$HT_2$ | Präfrontaler Cortex-Ratte | $^3$H-Ketanserin | Serotonin | 2400 |
| | | | Ketanserin | 0,1 |
| | | | Beispiel 1 | 1800 |

Beispiel 3 (Amphetaminpotenzierungs- und Antitetrabenazintest)

Die Verbindungen wurden auf antidepressive und das Sexualverhalten beeinflussende Wirkungen geprüft. Als Test für die antidepressive Wirkung diente der Amphetaminpotenzierungs- und der Antitetrabenazintest. Antidepressiv wirkende Substanzen potenzieren das Amphetamin-induzierte stereotype Verhalten bei der Ratte und antagonisieren die durch Tetrabenazin induzierte Ptosis bei der Maus. Der angegebene $ED_{50}$-Wert ist die Dosis, bei der das Verhalten nach Gabe von 2 mg/kg DL-Amphetaminsulfat i.v. um 50% verstärkt wird bzw. die durch 20 mg/kg i.p. Tetrabenazin induzierte Ptosis zu 50% reduziert wird.

Tabelle 2
Antidepressive Wirkung

| Testsystem | Tierart | $ED_{50}$ (mg/kg i.p.) |
|---|---|---|
| Amphetamin-Potenzierung | Ratte | 0,1 |
| Tetrabenazin-Antagonismus | Maus | 2,5 |

Literatur: J. L. Howard et al. in: Antidepressants: Neurochemical, behavioral and clinical perspectives Eds.: S. J. Enna et al., Raven Press, New York, S. 107–120, 1981.

Beispiel 4 (Einfluss auf Sexualverhalten)

Als Test für die das Sexualverhalten beeinflussende Wirkung diente die Messung der Ejakulationslatenz und die Anzahl der Kopulationsversuche. Die Werte geben die Zeit, die zwischen Zusammensetzen des männlichen und weiblichen Tieres und der ersten Ejakulation des Männchens vergeht, und die Anzahl der Kopulationsversuche an.

Tabelle 3
Wirkung auf das Sexualverhalten männlicher Ratten

| Dosis mg/kg i.p. | Anzahl der Tiere | Ejakulationslatenz (Sekunden) | | Anzahl der Kopulationsversuche | |
|---|---|---|---|---|---|
| | | Kontrolle | Bsp. 1 | Kontrolle | Bsp. 1 |
| 0,032 | 6 | 141 | 104 | 4,5 | 4 |
| 0,125 | 6 | 143 | 70** | 7,0 | 1* |
| 0,50 | 12 | 163 | 56** | 7,5 | 0*** |

* P – 0,05
** P – 0,025
*** P – 0,01 nach Mann-Whitney-Test

Literatur: Larsson K., Fuxe K., Everitt B.J., Holmgren M. und Södersten P. (1978). Sexual behaviour in male rats after intercerebral injection of 5–7-dihydrotryptamin Brain Research, 141, 293–303.

**Patentansprüche**

1. Tetrahydrobenzindole der allgemeinen Formel

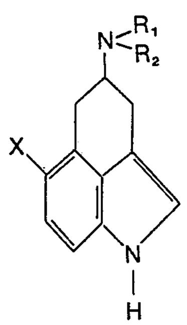

I

in welcher

$R_1$ und $R_2$ Wasserstoff oder $C_1$–$C_6$-Alkyl bedeuten oder zusammen mit dem Stickstoffatom einen gegebenenfalls durch 1 oder 2 $C_1$–$C_6$-Alkylgruppen substituierten heterocyclischen 5- oder 6-Ring bilden,

X für $OR_3$ oder $SR_3$ steht, wobei

$R_3$ Wasserstoff oder $C_1$–$C_4$-Alkyl bedeutet,

sowie deren Salze.

2. Tetrahydrobenzindole nach Anspruch 1, worin

$R_1$ und $R_2$ für einen geradkettigen oder verzweigten $C_1$–$C_4$-Alkylrest stehen oder $R_1$ und $R_2$ zusammen mit dem Stickstoffatom einen Pyrrolidin-, Piperidin-, 2,5-Dimethylpyrrolidin- oder 2,6-Dimethylpiperidinrest bilden und

X für $OCH_3$ oder $SCH_3$ steht,

sowie deren Salze.

3. Tetrahydrobenzindole nach den Ansprüchen 1 und 2, worin

$R_1$ und $R_2$ einen geradkettigen oder verzweigten $C_1$–$C_4$-Alkylrest und

X $OCH_3$ bedeutet,

sowie deren Salze.

4. Tetrahydrobenzindole nach Anspruch 1 der allgemeinen Formel

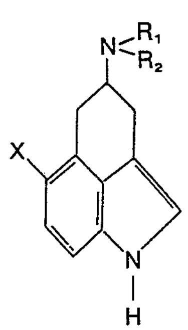

I

in welcher

$R_1$ und $R_2$ Wasserstoff oder $C_1$–$C_6$-Alkyl bedeuten oder zusammen mit dem Stickstoffatom einen gegebenenfalls durch 1 oder 2 $C_1$–$C_6$-Alkylgruppen substituierten heterocyclischen 5- oder 6-Ring bilden,

X für $OR_3$ oder $SR_3$ steht, wobei

$R_3$ Wasserstoff oder $C_1$–$C_4$-Alkyl bedeutet,

zur Verwendung bei der Bekämpfung von Krankheiten.

5. Arzneimittel, enthaltend mindestens eine Verbindung nach Anspruch 1 der allgemeinen Formel

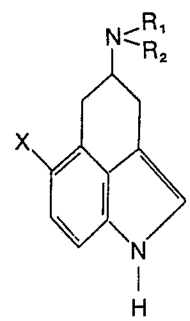

I

in welcher

$R_1$ und $R_2$ Wasserstoff oder $C_1$–$C_6$-Alkyl bedeuten oder zusammen mit dem Stickstoffatom einen gegebenenfalls durch 1 oder 2 $C_1$–$C_6$-Alkylgruppen substituierten heterocyclischen 5- oder 6-Ring bilden,

X für $OR_3$ oder $SR_3$ steht, wobei

$R_3$ Wasserstoff oder $C_1$–$C_4$-Alkyl bedeutet.

6. Verwendung von Verbindungen nach Anspruch 1 der allgemeinen Formel

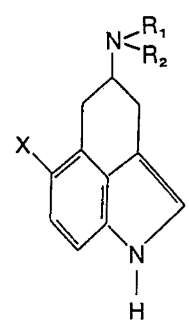

I

in welcher

$R_1$ und $R_2$ Wasserstoff oder $C_1$–$C_6$-Alkyl bedeuten oder zusammen mit dem Stickstoffatom einen gegebenenfalls durch 1 oder 2 $C_1$–$C_6$-Alkylgruppen substituierten heterocyclischen 5- oder 6-Ring bilden,

X für $OR_3$ oder $SR_3$ steht, wobei

$R_3$ Wasserstoff oder $C_1$–$C_4$-Alkyl bedeutet,

zur Herstellung von Arzneimitteln.

7. Verwendung nach Anspruch 6 zur Herstellung von Arzneimitteln zur Therapie von Krankheiten des zentralen Nervensytems, von kardiovaskulären Krankheiten, als Anxiolytika, Antidepressiva, Schlafmittel und als Mittel zur Potenzsteigerung.

8. Zubereitung enthaltend eine oder mehrere der Verbindungen nach Anspruch 1 der allgemeinen Formel

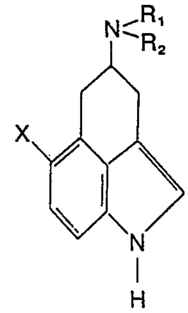

I

in welcher
$R_1$ und $R_2$ Wasserstoff oder $C_1$–$C_6$-Alkyl bedeuten oder zusammen mit dem Stickstoffatom einen gegebenenfalls durch 1 oder 2 $C_1$–$C_6$-Alkylgruppen substituierten heterocyclischen 5- oder 6-Ring bilden,
X für $OR_3$ oder $SR_3$ steht, wobei
$R_3$ Wasserstoff oder $C_1$–$C_4$-Alkyl bedeutet,
sowie inerte pharmazeutisch geeignete Trägerstoffe.

9. Verfahren zur Herstellung von Verbindungen nach Anspruch 1 der allgemeinen Formel

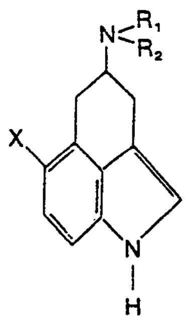

I

in welcher
$R_1$ und $R_2$ Wasserstoff oder $C_1$–$C_6$-Alkyl bedeuten oder zusammen mit dem Stickstoffatom einen gegebenenfalls durch 1 oder 2 $C_1$–$C_6$-Alkylgruppen substituierten heterocyclischen 5- oder 6-Ring bilden,
X für $OR_3$ oder $SR_3$ steht, wobei
$R_3$ Wasserstoff oder $C_1$–$C_4$-Alkyl bedeutet,
dadurch gekennzeichnet, dass man Verbindungen der allgemeinen Formel

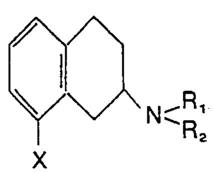

II

in der
$R_1$, $R_2$ und X die obengenannte Bedeutung haben, in para-Stellung zum Substituenten X nitriert, die entstandene Nitroverbindung mit Oxalsäureestern der Formel $R_4$–O–CO–CO–$OR_4$,
wobei
$R_3$ $CH_3$ oder $C_2H_5$ bedeutet,
zu Verbindungen der allgemeinen Formel

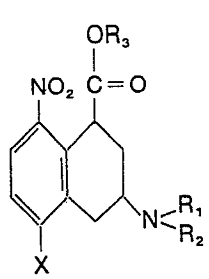

III

in der
$R_1$, $R_2$, $R_3$ und X die obengenannte Bedeutung haben,
acyliert, die Estergruppe verseift, die Nitrogruppe reduziert und anschliessend in einem oder mehreren Schritten cyclisiert und decarboxyliert.

10. Verfahren zur Herstellung von Verbindungen nach Anspruch 1 der allgemeinen Formel

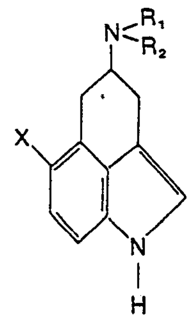

I

in welcher
$R_1$ und $R_2$ Wasserstoff oder $C_1$–$C_6$-Alkyl bedeuten oder zusammen mit dem Stickstoffatom einen gegebenenfall durch 1 oder 2 $C_1$–$C_6$-Alkylgruppen substituierten heterocyclischen 5- oder 6-Ring bilden,
X für $OR_3$ oder $SR_3$ steht, wobei
$R_3$ Wasserstoff oder $C_1$–$C_4$-Alkyl bedeutet,
dadurch gekennzeichnet, dass man Verbindungen der allgemeinen Formel

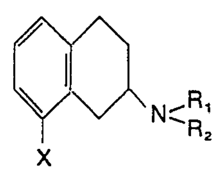

II

in der
$R_1$, $R_2$ und X die obengenannte Bedeutung haben,
in para-Stellung zum Substituenten X nitriert, zu den entsprechenden Aminen reduziert, dann die Isonitrile der Formel

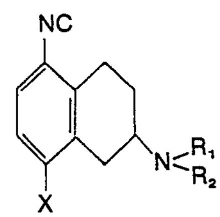

IV

in der
$R_1$, $R_2$ und X die obengenannte Bedeutung haben,
überführt und diese in Gegenwart einer starken Base cyclisiert.

**Claims**

1. Tetrahydrobenzindoles of the general formula

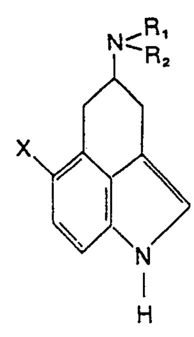

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl,

and salts thereof.

2. Tetrahydrobenzindoles according to Claim 1, wherein

$R_1$ and $R_2$ represent a straight chain or branched $C_1$–$C_4$-alkyl radical, or $R_1$ and $R_2$, together with the nitrogen atom, form a pyrrolidine, piperidine, 2,5-dimethylpyrrolidine or 2,6-dimethylpiperidine radical, and

X represents $OCH_3$ or $SCH_3$,

and salts thereof.

3. Tetrahydrobenzindoles according to Claims 1 and 2,

wherein

$R_1$ and $R_2$ denote a straight chain or branched $C_1$–$C_4$-alkyl radical and

X denotes $OCH_3$,

and salts thereof.

4. Tetrahydrobenzindoles according to Claim 1, of the general formula

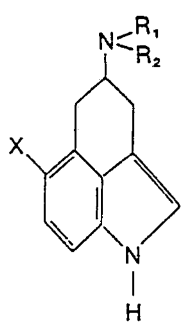

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl,

for use in combating diseases.

5. Medicaments containing at least one compound according to Claim 1, of the general formula

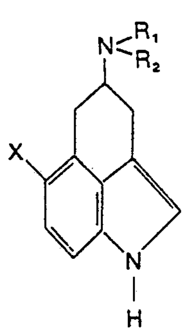

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl.

6. Use of compounds according to Claim 1, of the general formula

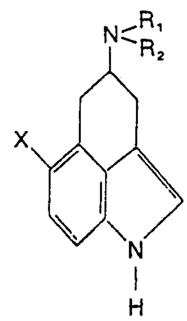

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl,

for the preparation of medicaments.

7. Use according to Claim 6 for the preparation of medicaments for the treatment of diseases of the central nervous system, cardiovascular diseases, as anxiolytic agents, antidepressant agents and soporific agents and as agents for increasing potency.

8. Formulation containing one or more of the compounds according to claim 1 of the general formula

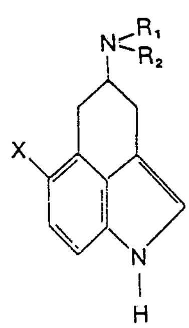

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl,

and inert pharmaceutically suitable excipients.

9. Process for the preparation of compounds according to Claim 1 of the general formula

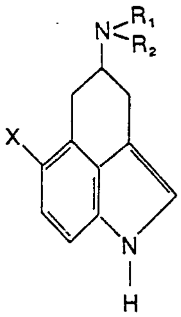

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl,

characterized in that compounds of the general formula

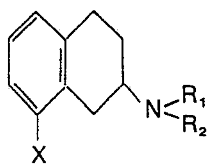

II

in which

$R_1$, $R_2$ and X have the abovementioned meaning,

are nitrated in the para-position relative to the substituent X, the resulting nitro compound is acylated with oxalic acid esters of the formula $R_4$–O–CO–CO–$OR_4$,

wherein

$R_3$ denotes $CH_3$ or $C_2H_5$,

to compounds of the general formula

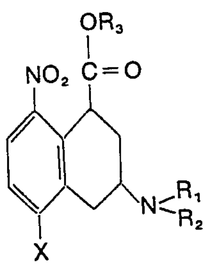

III

in which

$R_1$, $R_2$, $R_3$ and X have the abovementioned meaning, the ester group is hydrolysed and the nitro group is reduced, and the product is then cyclized and decarboxylated in one or more steps.

10. Process for the preparation of compounds according to Claim 1, of the general formula

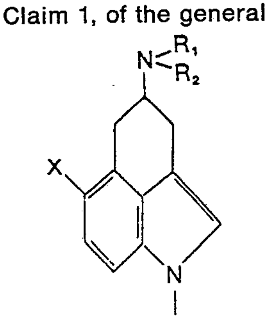

I

in which

$R_1$ and $R_2$ denote hydrogen or $C_1$–$C_6$-alkyl or, together with the nitrogen atom, form a heterocyclic 5-membered or 6-membered ring which is optionally substituted by 1 or 2 $C_1$–$C_6$-alkyl groups, and

X represents $OR_3$ or $SR_3$,

wherein

$R_3$ denotes hydrogen or $C_1$–$C_4$-alkyl,

characterized in that compounds of the general formula

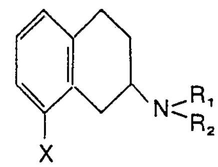

II

in which

$R_1$, $R_2$ and X have the abovementioned meaning, are nitrated in the para-position relative to the substituent X, the resulting compound is reduced to give the corresponding amines, and then these are converted to the isonitriles of the formula

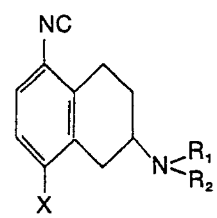

IV

in which

$R_1$m, $R_2$ and X have the abovementioned meaning, and the latter are cyclized in the presence of a strong base.

**Revendications**

1. Tétrahydrobenzindols de formule générale

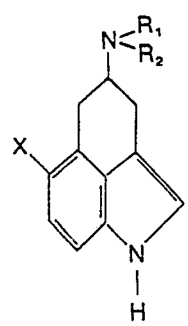

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle $C_1$–$C_6$

X représente $OR_3$ ou $SR_6$

$R_3$ signifiant de l'hydrogène ou un alcoyle $C_1$–$C_4$ de même que leurs sels.

2. Tétrahydrobenzindols selon la revendication 1 dans lesquels

$R_1$ et $R_2$ représentent un radical alcoyle en $C_1$–$C_4$ à chaîne droite ou ramifiée, ou bien $R_1$ et $R_2$,

conjointement avec l'atome d'azote, forment un radical pyrrolidine, pipéridine, 2,5-diméthylpyrrolidine ou 2,6-diméthylpipéridine et

X représente $OCH_3$ ou $SCH_3$

de même que leurs sels.

3. Tétrahydrobenzindols selon les revendications 1 et 2, dans lesquels

$R_1$ et $R_2$ signifient un radical alcoyle en $C_1$–$C_4$ à chaîne droite ou ramifiée et

X signifie $OCH_3$,

de même que leurs sels.

4. Tétrahydrobenzindols selon la revendication 1 de formule générale

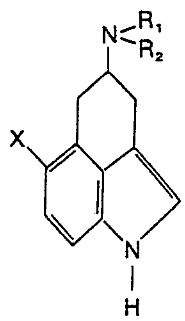

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle en $C_1$–$C_6$,

X représente $OR_3$ ou $SR_3$,

$R_3$ signifiant de l'hydrogène ou un alcoyle en $C_1$–$C_4$,

utilisés pour combattre les maladies.

5. Médicaments, contenant au moins un composé selon la revendication 1 de formule générale

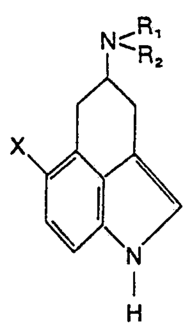

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle en $C_1$–$C_6$,

X représente $OR_3$ ou $SR_3$,

$R_3$ signifiant de l'hydrogène ou un alcoyle en $C_1$–$C_4$.

6. Utilisation des composés selon la revendication 1 de formule générale

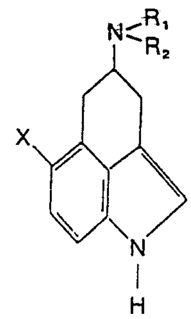

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle en $C_1$–$C_6$,

X représente $OR_3$ ou $SR_3$,

$R_3$ signifiant de l'hydrogène ou un alcoyle en $C_1$–$C_4$,

pour la fabrication de médicaments.

7. Utilisation selon la revendication 6 pour la fabrication de médicaments destinés à la thérapie des maladies du système nerveux central, des maladies cardiovasculaires, comme des anxiolytiques, antidépressifs, des somnifères et comme agents de potentialisation.

8. Préparation contenant un ou plusieurs des composés selon la revendication 1 de formule générale:

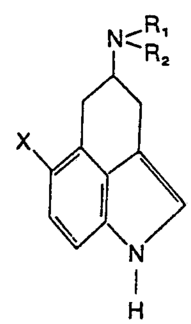

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle en $C_1$–$C_6$,

X représente $OR_3$ ou $SR_3$,

$R_3$ signifiant de l'hydrogène ou un alcoyle en $C_1$–$C_4$

de même que des matières de support inertes pharmaceutiquement appropriées.

9. Procédé de fabrication de composés selon la revendication 1 de formule générale:

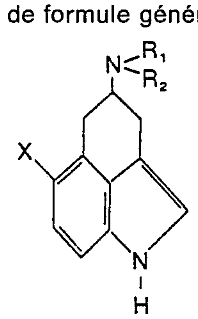

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle en $C_1$–$C_6$,

X représente $OR_3$ ou $SR_3$,

$R_3$ signifiant de l'hydrogène ou un alcoyle en $C_1$–$C_4$,

caractérisé en ce qu'on nitre des composés de formule générale

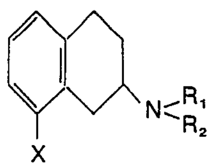

(II),

dans laquelle

$R_1$, $R_2$ et X ont la signification indiquée plus haut, en position para par rapport au substituant X, on acyle le nitrocomposé obtenu avec des esters oxaliques de formule $R_4$–O–CO–CO–$OR_4$, en l'occurrence

$R_3$ signifiant $CH_3$ ou $C_2H_5$,

pour donner des composés de formule générale

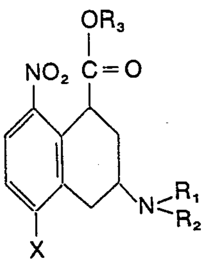

III

dans laquelle

$R_1$, $R_2$, $R_3$ et X ont la signification indiquée plus haut,

on saponifie le groupe ester, on réduit le groupe nitro et pour finir on cyclise et décarboxyle en un ou plusieurs stades.

10. Procédé de fabrication de composés selon la revendication1 de formule générale

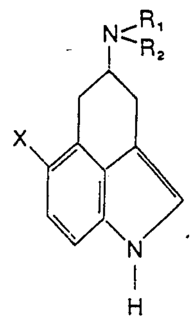

I

dans laquelle

$R_1$ et $R_2$ signifient de l'hydrogène ou un alcoyle en $C_1$–$C_6$, ou bien, conjointement avec l'atome d'azote, forment un noyau hétérocyclique à 5 ou 6 chaînons éventuellement substitué par 1 ou 2 groupes alcoyle en $C_1$–$C_6$,

X représente $OR_3$ ou $SR_3$,

$R_3$ signifiant de l'hydrogène ou un alcoyle en $C_1$–$C_4$,

caractérisé en ce qu'on nitre des composés de formule générale

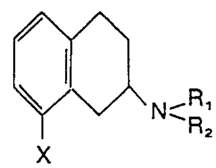

(II),

dans laquelle

$R_1$, $R_2$ et X ont la signification indiquée plus haut, en position para par rapport au substituant X, on réduit en les amines correspondantes puis on convertit celles-ci en isonitriles de formule

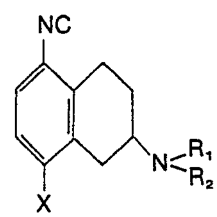

(IV),

dans laquelle

$R_1$, $R_2$ et X ont la signification citée plus haut, et en ce qu'on cyclise ces derniers en présence d'une base forte.